(19) Europäisches Patentamt
European Patent Office
Office européen des brevets

(11) Veröffentlichungsnummer: **0 149 131**
**A2**

(12) # EUROPÄISCHE PATENTANMELDUNG

(21) Anmeldenummer: **84115157.4**

(22) Anmeldetag: **11.12.84**

(51) Int. Cl.⁴: **C 07 D 471/04**
A 61 K 31/435
//C07D213/75, (C07D471/04, 235:00, 221:00)

(30) Priorität: **23.12.83 DE 3346602**

(43) Veröffentlichungstag der Anmeldung:
**24.07.85 Patentblatt 85/30**

(84) Benannte Vertragsstaaten:
**AT BE CH DE FR GB IT LI LU NL SE**

(71) Anmelder: **Dr. Karl Thomae GmbH**
**Postfach 1755**
**D-7950 Biberach (Riss)(DE)**

(72) Erfinder: **Mustel, Volhard, Dr. Dipl.-Chem.**
**Kapellenweg 7**
**D-7950 Biberach 1(DE)**

(72) Erfinder: **Hauel, Norbert, Dr. Dipl.-Chem.**
**Händelstrasse 12**
**D-7950 Biberach 1(DE)**

(72) Erfinder: **Heider, Joachim, Dr. Dipl.-Chem.**
**Am Hang 3**
**D-7951 Warthausen 1(DE)**

(72) Erfinder: **Reiffen, Manfred, Dr. Dipl.-Chem.**
**Matthias-Erzberger-Strasse 40**
**D-7950 Biberach 1(DE)**

(72) Erfinder: **van Meel, Jacobus, Dr.**
**Amriswilstrasse 7**
**D-7950 Biberach 1(DE)**

(72) Erfinder: **Diederen, Willi, Dr.**
**Haldenstrasse 1a**
**D-7950 Biberach 1(DE)**

(54) **Neue Imidazo4,5-cpyridin-one, deren Herstellung und diese Verbindungen enthaltende Arzneimittel.**

(57) Die Erfindung betrifft neue Imidazo[4,5-c]pyridin-one der allgemeinen Formel

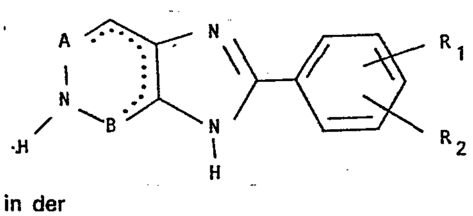

,(I)

in der
einer der Reste A oder B eine Methingruppe und der andere der Reste A oder B eine Carbonylgruppe,
$R_1$ eine Alkylmercapto-, Alkylsulfinyl- oder Alkylsulfonylgruppe und
$R_2$ eine Alkoxygruppe bedeuten, deren Tautomere und deren Säureadditionssalze, insbesondere deren physiologisch verträgliche Säureadditionssalze mit anorganischen oder organischen Säuren, welche wertvolle pharmakologische Eigenschaften aufweisen, insbesondere eine Wirkung auf die Kontraktilität des Herzmuskels.

Die neuen Verbindungen lassen sich nach für analoge Verbindungen bekannten Verfahren herstellen.

EP 0 149 131 A2

DR. KARL THOMAE GMBH
D-7950 Biberach 1

Case 5/897
Dr.Fl./Kp.



# Neue Imidazo[4,5-c]pyridin-one, deren Herstellung und diese Verbindungen enthaltende Arzneimittel

Die vorliegende Erfindung betrifft neue Imidazo[4,5-c]-pyridin-one der allgemeinen Formel

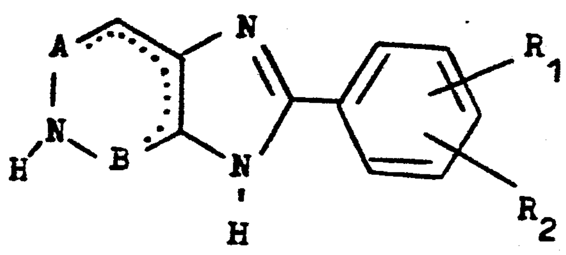

,(I)

deren Tautomere und deren Säureadditionssalze, insbesondere deren physiologisch verträgliche Säureadditionssalze mit anorganischen oder organischen Säuren, welche wertvolle pharmakologische Eigenschaften aufweisen, insbesondere eine Wirkung auf die Kontraktilität des Herzmuskels.

In der obigen allgemeinen Formel I bedeutet
einer der Reste A oder B eine Methingruppe und
der andere der Reste A oder B eine Carbonylgruppe,
$R_1$ eine Alkylmercapto-, Alkylsulfinyl- oder Alkylsulfonylgruppe und

$R_2$ eine Alkoxygruppe, wobei der Alkylteil jeweils 1 bis 3 Kohlenstoffatome enthalten kann.

Gegenstand der vorliegenden Erfindung sind somit die neuen Imidazo[4,5-c]pyridin-4-one und Imidazo[4,5-c]pyridin-6-one der obigen allgemeinen Formel I, deren Tautomere, deren Säureadditionssalze, insbesondere deren physiologisch verträgliche Säureadditionssalze mit anorganischen oder organischen Säuren, und diese Verbindungen enthaltende Arzneimittel sowie Verfahren zu ihrer Herstellung.

Für die bei der Definition der Reste $R_1$ und $R_2$ eingangs erwähnten Bedeutungen kommt beispielsweise

für $R_1$ die der Methylmercapto-, Äthylmercapto-, n-Propylmercapto-, Isopropylmercapto-, Methylsulfinyl-, Äthylsulfinyl-, n-Propylsulfinyl-, Isopropylsulfinyl-, Methylsulfonyl-, Äthylsulfonyl-, n-Propylsulfonyl- oder Isopropylsulfonylgruppe und

für $R_2$ die der Methoxy-, Äthoxy-, n-Propoxy- oder Isopropoxygruppe in Betracht.

Bevorzugte Verbindungen der allgemeinen Formel I sind jedoch diejenigen, in denen der Rest $R_1$ in 4-Stellung und der Rest $R_2$ in 2-Stellung des Phenylkerns steht.

Erfindungsgemäß erhält man die neuen Verbindungen nach folgenden Verfahren:

a) Cyclisierung einer gegebenenfalls im Reaktionsgemisch hergestellten Verbindung der allgemeinen Formel

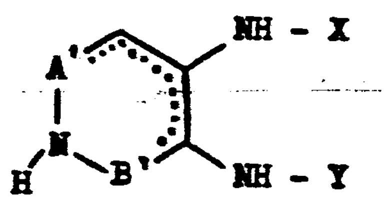

,(II)

in der

einer der Reste A' oder B' eine Methingruppe und der andere der Reste A' oder B' eine Carbonylgruppe oder eine Alkoxymethingruppe, wobei die Alkoxygruppe 1 bis 3 Kohlenstoffatome enthalten kann,

einer der Reste X oder Y ein Wasserstoffatom und der andere der beiden Reste X und Y oder beide Reste X und Y eine Gruppe der Formel

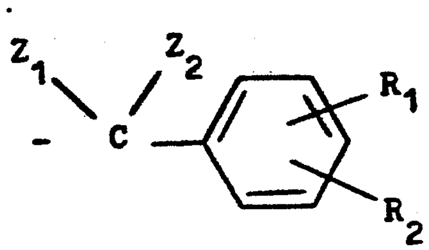

darstellen, in der

$R_1$ und $R_2$ wie eingangs definiert sind,

$Z_1$ und $Z_2$, die gleich oder verschieden sein können, Halogenatome, gegebenenfalls substituierte Aminogruppen oder gegebenenfalls durch niedere Alkylgruppen substituierte Hydroxy- oder Mercaptogruppen oder

$Z_1$ und $Z_2$, zusammen ein Sauerstoff- oder Schwefelatom, eine gegebenenfalls durch eine Alkylgruppe mit 1 bis 3 Kohlenstoffatomen mono- oder disubstituierte Iminogruppe, eine Alkylendioxy- oder Alkylendithiogruppe mit jeweils 2 oder 3 Kohlenstoffatomen bedeuten.

Die Cyclisierung wird zweckmäßigerweise in einem Lösungsmittel oder Lösungsmittelgemisch wie Ethanol, Isopropanol, Eisessig, Benzol, Chlorbenzol, Toluol, Xylol, Glycol, Glycolmonomethylether, Diethylenglycoldimethylether, Sulfolan, Dimethylformamid, Tetralin oder in einem Überschuß des zur Herstellung der Verbindung der allgemeinen Formel II verwendeten Acylierungsmittel, z.B. in dem entsprechenden Nitril,

Anhydrid, Säurehalogenid, Ester, Amid oder Methojodid, beispielsweise bei Temperaturen zwischen 0 und 250°C, vorzugsweise jedoch bei der Siedetemperatur des Reaktionsgemisches, gegebenenfalls in Gegenwart eines Kondensationsmittels wie Phosphoroxychlorid, Thionylchlorid, Sulfurylchlorid, Schwefelsäure, p-Toluolsulfonsäure, Salzsäure, Phosphorsäure, Polyphosphorsäure, Essigsäureanhydrid oder gegebenenfalls auch in Gegenwart einer Base wie Kaliumäthylat oder Kalium-tert.butylat durchgeführt. Die Cyclisierung kann jedoch auch ohne Lösungsmittel und/oder Kondensationsmittel durchgeführt werden.

Bei Einsatz einer Verbindung der allgemeinen Formel II, in der einer der Reste A' oder B' eine Alkoxymethingruppe darstellt, kann die Alkoxymethingruppe während der Umsetzung oder auch nachträglich, z.B. durch saure oder alkalische Hydrolyse, in eine Carbonylgruppe übergeführt werden. Hierbei wird die nachträgliche Hydrolyse vorzugsweise in Gegenwart einer Säure wie Schwefelsäure, Salzsäure, Phosphorsäure oder p-Toluolsulfonsäure bei Temperaturen zwischen 80 und 120°C durchgeführt.

b) Zur Herstellung von Verbindungen der allgemeinen Formel I, in der $R_1$ eine Alkylsulfinyl- oder Alkylsulfonylgruppe darstellt:

Oxidation einer Verbindung der allgemeinen Formel

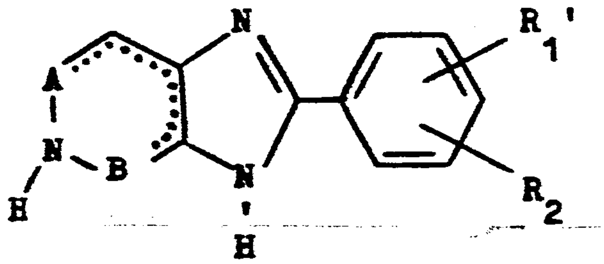

,(III)

in der
$R_2$, A und B wie eingangs definiert sind und
$R_1$' eine Alkylmercapto- oder Alkylsulfinylgruppe mit jeweils 1 bis 3 Kohlenstoffatomen darstellt.

Die Oxidation wird vorzugsweise in einem Lösungsmittel oder Lösungsmittelgemisch, z.B. in Wasser, Wasser/Pyridin, Aceton, Eisessig, verdünnter Schwefelsäure oder Trifluoressigsäure, je nach dem verwendeten Oxidationsmittel zweckmäßigerweise bei Temperaturen zwischen -80 und 100°C durchgeführt.

Zur Herstellung einer Alkylsulfinylverbindung der allgemeinen Formel I wird die Oxidation zweckmäßigerweise mit einem Äquivalent des verwendeten Oxidationsmittels durchgeführt, z.B. mit Wasserstoffperoxid in Eisessig, Trifluoressigsäure oder Ameisensäure bei 0 bis 20°C oder in Aceton bei 0 bis 60°C, mit einer Persäure wie Perameisensäure in Eisessig oder Trifluoressigsäure bei 0 bis 50°C oder mit m-Chlorperbenzoesäure in Methylenchlorid oder Chloroform bei -20 bis 60°C, mit Natriummetaperjodat in wässrigem Methanol oder Äthanol bei -15 bis 25°C, mit Brom in Eisessig oder wässriger Essigsäure, mit N-Brom-succinimid in Äthanol, mit tert.Butyl-hyprochlorit in Methanol bei -80 bis -30°C, mit Jodbenzodichlorid in wässrigem Pyridin bei 0 bis 50°C, mit Salpetersäure in Eisessig bei 0 bis 20°C, mit Chromsäure in Eisessig oder in Aceton bei 0 bis 20°C und mit Sulfurylchlorid in Methylenchlorid bei -70°C, der hierbei erhaltene Thioäther-Chlor-Komplex wird zweckmäßigerweise mit wäßrigem Äthanol hydrolysiert.

Zur Herstellung einer Alkylsulfonylverbindung der allgemeinen Formel I wird die Oxidation zweckmäßigerweise mit einem bzw. mit zwei oder mehr Äquivalenten des verwendeten Oxidationsmittels durchgeführt, z.B. mit Wasserstoffperoxid in Eisessig, Trifluoressigsäure oder in Ameisensäure bei 20 bis

100°C oder in Aceton bei 0 bis 60°C, mit einer Persäure wie Perameisensäure oder m-Chlorperbenzoesäure in Eisessig, Trifluoressigsäure, Methylenchlorid oder Chloroform bei Temperaturen zwischen 0 und 60°C, mit Salpetersäure in Eisessig bei 0 bis 20°C, mit Chromsäure oder Kaliumpermanganat in Eisessig, Wasser/Schwefelsäure oder in Aceton bei 0 bis 20°C.

Die erhaltenen Verbindungen der allgemeinen Formel I können anschließend gewünschtenfalls in ihre physiologisch verträglichen Säureadditionssalze mit anorganischen oder organischen Säuren übergeführt werden. Als Säuren kommen hierfür beispielsweise Salzsäure, Bromwasserstoffsäure, Schwefelsäure, Phosphorsäure, Furmarsäure, Bernsteinsäure, Weinsäure, Zitronensäure, Milchsäure, Maleinsäure oder Methansulfonsäure in Betracht.

Die als Ausgangsstoffe verwendeten Verbindungen der allgemeinen Formeln II und III sind teilweise literaturbekannt bzw. erhält man sie nach literaturbekannten Verfahren. So erhält man beispielsweise die als Ausgangsstoffe verwendeten Verbindungen der allgemeinen Formel II durch Acylierung der entsprechenden o-Diaminoverbindungen bzw. durch Reduktion der entsprechenden Acylamino-nitro-Verbindungen sowie die Verbindungen der allgemeinen Formel III durch anschließenden Ringschluß (siehe BE-PS 810.545 und EP-A-0.024.290).

Wie bereits eingangs erwähnt weisen die neuen Verbindungen der allgemeinen Formel I, deren Tautomere und deren physiologisch verträgliche Säureadditionssalze überlegene pharmakologische Eigenschaften auf, insbesondere eine Blutdruck- und/oder positiv inotrope Wirkung.

Beispielweise wurde die Verbindung

A = 2-(2-Methoxy-4-methylmercapto-phenyl)-5H-imidazo[4,5-c]-pyridin-6-on

auf ihre biologischen Eigenschaften wie folgt untersucht:

Bestimmung der Blutdruckwirkung und der positiv inotropen Wirkung an der narkotisierten Katze

Die Untersuchungen wurden an Katzen durchgeführt, die mit Pentobarbital-Natrium (40 mg/kg i.p.) narkotisiert waren. Die Tiere atmeten spontan. Der arterielle Blutdruck wurde in der Aorta abdominalis mit einem Statham-Druckwandler (P 23 Dc) gemessen. Für die Erfassung der positiv inotropen Wirkung wurde mit einem Kathetertipmanometer (Millar PC-350 A) der Druck in der linken Herzkammer gemessen. Daraus wurde der Kontraktilitätsparamter $dp/dt_{max}$ mittels eines Analog-differenzierers gewonnen. Die zu untersuchenden Substanzen wurden in eine Vena femoralis injiziert. Als Lösungsmittel diente Polydiol 200. Jede Substanz wurde an mindestens 3 Katzen geprüft, Dosis 2 mg/kg i.v..

Die nachfolgende Tabelle enthält die Mittelwerte:

| Substanz | Zunahme von $dp/dt_{max}$ in % | Blutdruckwirkung in mm Hg | Wirkungsdauer (Halbwertszeit) in Minuten |
|---|---|---|---|
| A | + 143 | + 41/+ 24 | 27 |

Die neuen Verbindungen sind gut verträglich, so konnten bei der Untersuchung der Substanz A keinerlei herztoxische Wirkungen bzw. Kreislaufschäden beobachtet werden.

Aufgrund ihrer pharmakologischen Eigenschaften eignen sich die erfindungsgemäß hergestellten Verbindungen der allgemeinen Formel I sowie deren physiologisch verträgliche Säureadditionssalze zur Behandlung von Herzinsuffizienzen unterschiedlicher Genese, da sie die Kontraktionskraft des Herzens steigern.

Hierzu lassen sich die neuen Verbindungen sowie deren physiologisch verträgliche Säureadditionssalze, gegebenenfalls in Kombination mit anderen Wirksubstanzen, in die üblichen pharmazeutischen Anwendungsformen wie Tabletten, Dragées, Pulver, Suppositorien, Suspensionen, Ampullen oder Tropfen einarbeiten. Die Einzeldosis beträgt hierbei 1-4 x täglich 0,1 - 5 mg/kg Körpergewicht, vorzugsweise jedoch 0,5 - 2,0 mg/kg Körpergewicht.

Die nachfolgenden Beispiele sollen die Erfindung näher erläutern:

Beispiel 1

2-(2-Methoxy-4-methylmercapto-phenyl)-5H-imidazo[4,5-c]-pyridin-6-on

a) 4-(2-Methoxy-4-methylmercapto-benzoylamino)-5-nitro-1H-pyridin-2-on

0,45 g 4-Amino-5-nitro-1H-pyridin-2-on und 0,87 g 2-Methoxy-4-methylmercapto-benzoylchlorid werden in 10 ml Chlorbenzol 8 Stunden zum Rückfluß erhitzt. Das ausgefallene Produkt wird abgesaugt, mit Methylenchlorid gewaschen und als Rohprodukt weiterverwendet.
Ausbeute: 0,72 g (57 % der Theorie).

b) 2-(2-Methoxy-4-methylmercapto-phenyl)-5H-imidazo[4,5-c]-pyridin-6-on

0,6 g rohes 4-(2-Methoxy-4-methylmercapto-benzoylamino)-5-nitro-1H-pyridin-2-on werden in 10 ml Eisessig gelöst, mit zwei Spatelspitzen Eisenpulver versetzt und zunächst 30 Minuten bei Raumtemperatur gerührt. Anschließend wird noch 20 Minuten zum Rückfluß erhitzt, filtriert und das Filtrat im Vakuum eingedampft. Das erhaltene Produkt wird durch Chromatographie an Kieselgel (Elutionsmittel: Methylenchlorid/Ethanol = 100:2 bis 100:15) gereinigt.
Ausbeute: 0,13 g (22 % der Theorie),
Schmelzpunkt: 236-238°C.

Beispiel 2

2-(2-Methoxy-4-methylmercapto-phenyl)-5H-imidazo[4,5-c]-pyridin-4-on

0,88 g 2-Methoxy-3,4-diamino-pyridin-hydrochlorid und 1,08 g

2-Methoxy-4-methylmercapto-benzoylchlorid werden innig vermischt und in 120°C warme Polyphosphorsäure eingerührt. Man hält noch eine Stunde bei dieser Temperatur, gießt auf Wasser, stellt ammoniakalisch und reinigt den ausgefallenen Niederschlag über eine Kieselgelsäule (Elutionsmittel: Methylenchlorid/Methanol = 100:10).
Ausbeute: 0,22 g (15 % der Theorie),
Schmelzpunkt: 243-245°C.

## Beispiel 3

### 2-(2-Methoxy-4-methylsulfinyl-phenyl)-5H-imidazo[4,5-c]-pyridin-4-on

0,3 g 2-(2-Methoxy-4-methylmercapto-phenyl)-5H-imidazo-[4,5-c]pyridin-4-on werden in 10 ml 50%iger Essigsäure suspendiert und dazu 0,2 g wasserfreies Natriumacetat gegeben. Das Gemisch wird unter Rühren tropfenweise mit 0,15 g Brom, gelöst in 2 ml Eisessig, versetzt. Nach Beendigung der Reaktion wird auf Eis gegossen und der ausgefallene Niederschlag aus Ethanol umkristallisiert.
Schmelzpunkt: 267-272°C (Zersetzung)

## Beispiel 4

### 2-(2-Methoxy-4-methylsulfonyl-phenyl)-5H-imidazo[4,5-c]-pyridin-4-on

0,14 g 2-(2-Methoxy-4-methylmercapto-phenyl)-5H-imidazo-[4,5-c]pyridin-4-on werden in 10 ml Eisessig gelöst, mit 0,5 ml 30%igem Wasserstoffperoxid versetzt und 2 Stunden auf 40-50°C erwärmt. Die Reaktionsmischung wird weitgehend eingedampft, mit Eiswasser versetzt und der ausgefallene Niederschlag abgesaugt.
Schmelzpunkt: 290°C (Zersetzung)

Beispiel 5

2-(2-Methoxy-4-methylsulfinyl-phenyl)-5H-imidazo[4,5-c]-pyridin-6-on

Hergestellt analog Beispiel 3 aus 2-(2-Methoxy-4-methylmercapto-phenyl)-5H-imidazo[4,5-c]pyridin-6-on

Beispiel 6

2-(2-Methoxy-4-methylsulfonyl-phenyl)-5H-imidazo[4,5-c]pyridin-6-on

Hergestellt analog Beispiel 4 aus 2-(2-Methoxy-4-methylmercapto-phenyl)-5H-imidazo[4,5-c]pyridin-6-on.

Beispiel 7

2-(2-Propoxy-4-methylmercapto-phenyl)-5H-imidazo[4,5-c]pyridin-4-on-hydrochlorid

Hergestellt analog Beispiel 2 aus 2-Propoxy-4-methylmercapto-benzoylchlorid und 2-Methoxy-3,4-diamino-pyridin-hydrochlorid.

Schmelzpunkt: über 260°C

| | | | | | | |
|---|---|---|---|---|---|---|
| Ber.: | C | 54,62 | H | 5,16 | N | 11,94 |
| Gef.: | | 54,60 | | 5,45 | | 11,95 |

Beispiel 8

2-(2-Methoxy-4-methylsulfinyl-phenyl)-5H-imidazo[4,5-c]-pyridin-4-on und

2-(2-Methoxy-4-methylsulfonyl-phenyl)-5H-imidazo[4,5-c]-pyridin-4-on

---

0,6 g 2-(2-Methoxy-4-methylmercapto-phenyl)-5H-imidazo-[4,5-c]pyridin-4-on werden in 5 ml Eisessig verrührt, mit 0,5 ml 35%igem Wasserstoffperoxid versetzt und weitere eineinhalb Stunden bei Raumtemperatur gerührt. Man verdünnt mit Wasser, neutralisiert mit konzentriertem Ammoniak, dampft ein und reinigt durch Chromatographie über Kieselgel (Elutionsmittel = Methylenchlorid/Ethanol = 9:1). Ausbeute der Sulfinylverbindung: 0,18 g (28 % der Theorie), Schmelzpunkt: 267-272°C (Zersetzung)

Ausbeute der Sulfonylverbindung: 0,21 g (32 % der Theorie), Schmelzpunkt: 290°C (Zersetzung)

Beispiel A

Tabletten zu 100 mg 2-(2-Methoxy-4-methylmercapto-phenyl)-5H-imidazo[4,5-c]pyridin-6-on

Zusammensetzung
1 Tablette enthält:

| | |
|---|---|
| Wirksubstanz | 100,0 mg |
| Milchzucker | 50,0 mg |
| Polyvinylpyrrolidon | 5,0 mg |
| Carboxymethylcellulose | 19,0 mg |
| Magnesiumstearat | 1,0 mg |
| | 175,0 mg |

Feuchtsiebung: 1,5 mm
Trocknen: Umlufttrockenschrank 50°C
Trockensiebung: 1 mm
Dem Granulat die restlichen Hilfsstoffe zumischen und Endmischung zu Tabletten verpressen.
Tablettengewicht: 175 mg
Stempel: 8 mm

Beispiel B

Dragées zu 50 mg 2-(2-Methoxy-4-methylmercapto-phenyl)-5H-imidazo[4,5-c]pyridin-6-on

Zusammensetzung:
1 Dragéekern enthält:

| | |
|---|---|
| Wirksubstanz | 50,0 mg |
| Maisstärke getr. | 20,0 mg |
| Lösliche Stärke | 2,0 mg |
| Carboxymethylcellulose | 7,0 mg |
| Magnesiumstearat | 1,0 mg |
| | 80,0 mg |

Wirkstoff und Stärke mit wäßriger Lösung der löslichen Stärke gleichmäßig befeuchten.

Feuchtsiebung: 1,0 mm
Trockensiebung: 1,0 mm,
Trocknung: 50°C im Umlufttrockenschrank

Granulat und restliche Hilfsstoffe mischen und zu Kernen verpressen.

Kerngewicht: 80 mg
Stempel: 6 mm
Wölbungsradius: 5 mm

Die fertigen Kerne werden auf übliche Weise mit einem Zuckerüberzug im Dragierkessel versehen.

Dragéegewicht: 120 mg

## Beispiel C

**Suppositorien zu 75 mg 2-(2-Methoxy-4-methylmercapto-phenyl)-imidazo[4,5-c]pyridin-6-on**

1 Zäpfchen enthält:

| | |
|---|---|
| Wirksubstanz | 75,0 mg |
| Zäpfchenmasse (z.B. Witepsol H 19 und Witepsol W 45) | 1 625,0 mg |
| | 1 700,0 mg |

**Herstellungsverfahren:**

Die Zäpfchenmasse wird geschmolzen. Bei 38°C wird die gemahlene Wirksubstanz in der Schmelze homogen dispergiert. Es wird auf 35°C abgekühlt und in vorgekühlte Suppositorienformen ausgegossen.

Zäpfchengewicht: 1,7 g

Beispiel D

Ampullen zu 50 mg 2-(2-Methoxy-4-methylmercapto-phenyl)-imidazo[4,5-c]pyridin-6-on

1 Ampulle enthält:

| | |
|---|---|
| Wirksubstanz | 50,0 mg |
| Ethoxylierte Hydroxystearinsäure | 750,0 mg |
| 1,2-Propylenglykol | 1000,0 mg |
| Dest. Wasser ad | 5,0 ml |

Herstellungsverfahren:

Die Wirksubstanz wird in 1,2-Propylenglykol und ethoxylierter Hydroxystearinsäure gelöst, dann mit Wasser auf das angegebene Volumen aufgefüllt und steril filtriert.

Abfüllung: in Ampullen zu 5 ml

Sterilisation: 20 Minuten bei 120°C

Beispiel E

Tropfen zu 100 mg 2-(2-Methoxy-4-methylmercapto-phenyl)-imidazo[4,5-c]pyridin-6-on

| | | |
|---|---|---|
| Wirksubstanz | | 1,0 g |
| p-Oxybenzoesäuremethylester | | 0,035 g |
| p-Oxybenzoesäurepropylester | | 0,015 g |
| Anisöl | | 0,05 g |
| Menthol | | 0,06 g |
| Saccharin-Natrium | | 1,0 g |
| Glycerin | | 10,0 g |
| Ethanol | | 40,0 g |
| Dest. Wasser | ad | 100,0 ml |

Herstellungsverfahren:

Die Benzoesäureester werden in Ethanol gelöst und anschließend das Anisöl und das Menthol zugegeben. Dann wird die Wirksubstanz, Glycerin und Saccharin-Natrium im Wasser gelöst zugegeben. Die Lösung wird anschließend klar filtriert.

## Patentansprüche

1. Imidazo[4,5-c]pyridin-one der allgemeinen Formel

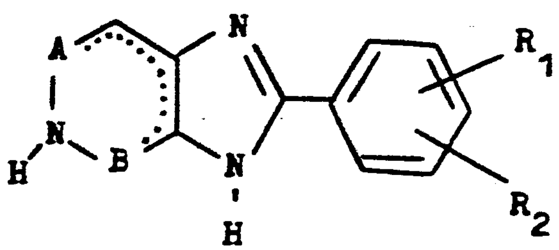

,(I)

in der einer der Reste A oder B eine Methingruppe und der andere der Reste A oder B eine Carbonylgruppe, $R_1$ eine Alkylmercapto-, Alkylsulfinyl- oder Alkylsulfonylgruppe und $R_2$ eine Alkoxygruppe, wobei der Alkylteil jeweils 1 bis 3 Kohlenstoffatome enthalten kann, bedeuten, deren Tautomere und deren Säureadditionssalze.

2. Imidazo[4,5-c]pyridin-one der allgemeinen Formel I gemäß Anspruch 1, in der

A, B, $R_1$ und $R_2$ wie im Anspruch 2 definiert sind und $R_1$ in 4-Stellung und $R_2$ in 2-Stellung des Phenylkerns stehen, deren Tautomere und deren Säureadditionssalze.

3. 2-(2-Methoxy-4-methylmercapto-phenyl)-5H-imidazo[4,5-c]-pyridin-6-on, dessen Tautomere und dessen Säureadditionssalze.

4. Physiologisch verträgliche Säureadditionssalze der Verbindungen gemäß den Ansprüchen 1-3.

5. Arzneimittel, enthaltend eine Verbindung gemäß den Ansprüchen 1-3 oder ein physiologisch verträgliches Säureadditionssalz gemäß Anspruch 4 neben einem oder mehreren inerten Trägerstoffen und/oder Verdünnungsmitteln.

6. Verwendung einer Verbindung gemäß den Ansprüchen 1-3 oder eines physiologisch verträglichen Säureadditionssalzes gemäß Anspruch 4 zur Behandlung von Herzinsuffizienzen.

7. Verfahren zur Herstellung eines Arzneimittels gemäß Anspruch 5, dadurch gekennzeichnet, daß auf nichtchemischem Wege eine Verbindung gemäß den Ansprüchen 1-3 oder ein physiologisch verträgliches Säureadditionssalz gemäß Anspruch 4 in einen oder mehreren inerten Trägerstoffen und/oder Verdünnungsmitteln eingearbeitet wird.

8. Verfahren zur Herstellung der Verbindungen gemäß den Ansprüchen 1-4 dadurch gekennzeichnet, daß

a) eine gegebenenfalls im Reaktionsgemisch hergestellte Verbindung der allgemeinen Formel

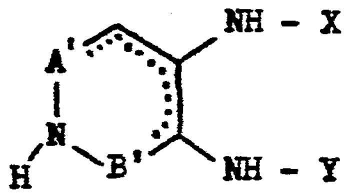

,(II)

in der
einer der Reste A' oder B' eine Methingruppe und der andere der Reste A' oder B' eine Carbonylgruppe oder eine Alkoxymethingruppe, wobei die Alkoxygruppe 1 bis 3 Kohlenstoffatome enthalten kann,
einer der Reste X oder Y ein Wasserstoffatom und der andere der beiden Reste X und Y oder beide Reste X und Y eine Gruppe der Formel

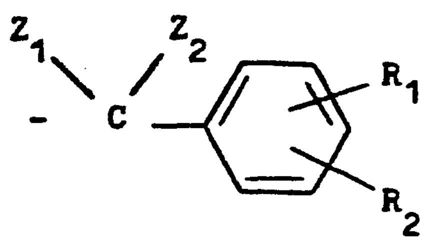

darstellen, in der

$R_1$ und $R_2$ im Anspruch 1 definiert sind,
$Z_1$ und $Z_2$, die gleich oder verschieden sein können, Halogenatome, gegebenenfalls substituierte Aminogruppen oder gegebenenfalls durch niedere Alkylgruppen substituierte Hydroxy- oder Mercaptogruppen oder

$Z_1$ und $Z_2$, zusammen ein Sauerstoff- oder Schwefelatom, eine gegebenenfalls durch eine Alkylgruppe mit 1 bis 3 Kohlenstoffatomen mono- oder disubstituierte Iminogruppe, eine Alkylendioxy- oder Alkylendithiogruppe mit jeweils 2 oder 3 Kohlenstoffatomen bedeuten, cyclisiert und, falls eine Verbindung der allgemeinen Formel I erhalten wird, in der einer der Reste A oder B eine Alkoxymethingruppe darstellt, diese hydrolysiert wird oder

b) zur Herstellung von Verbindungen der allgemeinen Formel I, in der $R_1$ eine Alkylsulfinyl- oder Alkylsulfonylgruppe darstellt, eine Verbindung der allgemeinen Formel

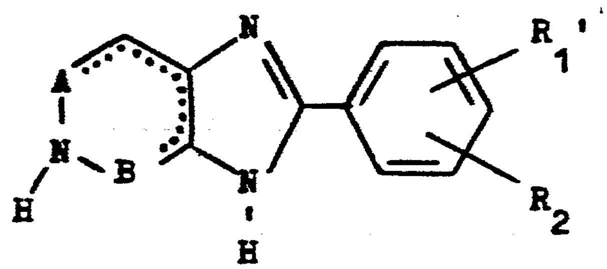

,(III)

in der
$R_2$, A und B wie im Anspruch 1 definiert sind und
$R_1'$ eine Alkylmercapto- oder Alkylsulfinylgruppe mit jeweils 1 bis 3 Kohlenstoffatomen darstellt, oxidiert wird und gewünschtenfalls anschließend eine so erhaltene Verbindung der allgemeinen Formel I in ihr Säureadditionssalz, insbesondere in ihr physiologisch verträgliches Säureadditionssalz mit einer anorganischen oder organischen Säure, übergeführt wird.

9. Verfahren nach Anspruch 8, dadurch gekennzeichnet, daß die Umsetzung in einem Lösungsmittel durchgeführt wird.

10. Verfahren nach den Ansprüchen 8a und 9, dadurch gekennzeichnet, daß eine in einer verwendeten Verbindung der allgemeinen Formel IIb enthaltene Alkoxymethingruppe während der Umsetzung oder nachträglich in eine Carbonylgruppe übergeführt wird.